# NEW EUROPEAN PATENT SPECIFICATION

(11) **EP 0 317 521 B2**
(45) Date of publication and mention of the opposition decision: **11.12.1996**
(45) Mention of the grant of the patent: 18.09.1991
(21) Application number: 88830461.5
(22) Date of filing: 28.10.1988
(51) Int. Cl.: A61L 2/18, A61C 19/00

(54) **A process and device for sterilization of the spray and/or air supply lines to medical instruments, in particular dental surgery instruments**
Verfahren und Einrichtung zur Sterilisation des Zerstäubers und/oder der Luftzufuhr von medizinischen Instrumenten, insbesondere von zahnärztlichen Instrumenten
Procédé et dispositif pour la stétilisation du pulvérisateur et/ou de la conduite d'air des instruments médicaux, en particulier des instruments de chirurgie dentaire

(30) Priority: 18.11.1987 IT 369987; 22.06.1988 IT 351488
(43) Date of publication of application: 24.05.1989
(73) Proprietor: CASTELLINI S.p.A., I-40013 Castelmaggiore (Bologna) (IT)
(72) Inventor: Castellini, Franco, I-40135 Bologna (IT); Pignattini, Giorgio, I-40127 Bologna (IT); Lanzarini, Giuliano, I-40138 Bologna (IT); Frisullo, Rocco, I-40013 Castelmaggiore (Bologna) (IT)
(74) Representative: Lanzoni, Luciano

(56) References cited:
- EP-A- 0 233 847
- DE-A- 3 246 266
- DE-A- 3 611 327
- DE-A- 3 635 568
- US-A- 4 752 444

## Description

The invention relates to a process and device for sterilization of the spray and/or air supply lines to power driven medical instruments, in particular dental surgery instruments.

German patent DE-OS 2836532 disdoses a process, and apparatus, for sterilizing the handgrips of medical or dental surgery instruments. The process is essentially one of subjecting fluid propellants and/or coolants to the germicidal action of an oxidizing medium, for example anode oxidation or microwave radiation.

Such a process is beset by problems of efficiency, however, especially as regards sterilization of the fluids when passing through the oxidizing medium, or of safety in the case of radiation, given that microwave sources require appropriate screening. The apparatus itself is similarly beset by problems stemming from the high quantity of energy required for its operation, the effect of which is to limit the scope of application.

A different type of device for sterilizing medical instruments, and for dental surgery instruments in particular, is disclosed in another German patent DE-OS 3028550. This consists in a container, to which liquid sterilizer is supplied, and a metering pump by which the liquid sterilizer is forced into the spray circuit of each instrument, according to the quantity of water required by the instrument during operation. To this end, the metering pump is interlocked to control media capable of identifying which instrument is activated, and determining the quantity of liquid sterilizer to be pumped into the spray circuit, likewise according to the quantity of water utilized in operation.

This device is certainly effective, though only in the case of a continuous sterilization of the water supplied to instruments, at low rates of flow, and only for a limited sterilizing action, given that the disinfectant is pumped into the spray circuit while the instruments are in use ; more exactly, the liquid sterilizer mingles with the water sent into the instrument, and thus enters into contact with the internal surfaces of the human body (the mouth, in the instance of dental surgery instruments), and accordingly, the liquid sterilizer and/or its level of concentration in the spray must be formulated and/or calculated to ensure that there is no health risk. As most of the liquid sterilizers currently in use are acids, and toxic, they must be diluted considerably in this type of situation, and thus lose a great deal of their germicidal properties. Further apparatus for the sterilization of medical instruments is disdosed, together with a relative process, in DE-OS 3246266. In this instance, the spray circuit serving the instruments is isolated by a main shut-off valve and the individual on-off valve at each instrument opened up, whereupon a liquid sterilizer is pumped into the supply lines downstream of the main shut-off valve ; after being allowed to circulate for a prescribed period, the flow of sterilizer is stopped and the main shut-off valve re-opened to allow water back into the rest of the circuit downstream so that all traces of the soiled liquid can be flushed out, the individual instrument valves remaining open for long enough to ensure its complete explusion.

With this type of apparatus, it becomes possible to sterilize with much stronger solutions, and thus to greater effect, since the process is implemented at a time when the instruments are not in use, say, at the end of the working day, or of the working week. Neither is this apparatus wholly without drawbacks, however : when flushing out the circuit for example, a considerable amount of water (and of time) is required to ensure that no traces of the sterilizer remain, however minute ; also, a not inconsiderable quantity of liquid sterilizer is required to fill up the supply lines completely.

A further drawback of the apparatus in question is its complexity. To enable sterilization, in fact, the on-off valves of the single instruments must be held open, whereas these valves are of a normally-closed type designed to open, singly, when the relative instrument is in use. More exactly, the valve can be opened only by removing the instrument from its holder, for obvious expediency ; however, in the event that not all of the on-off valves are opened up, due to an oversight or for any other reason, the instrument or instruments remaining 'off' will undergo no sterilizing action whatever.

Another problem with the same apparatus stems from the fact that sterilizer is caused to flow through the on-off valves of the instruments, and thus to occupy them for a given duration ; the inevitable result is that the valves are prone to damage from the aggressive properties of the fluid, as well as from the risk that the valve housing may not be flushed absolutely clean following sterilization.

Accordingly, the object of the invention is to overcome the drawbacks described above.

The stated object is achieved with a process and a device as described in the appended claims; such a process involves directing liquid sterilizer into the spray and/or air supply lines downstream of the relative on-off valves, the valves being closed, and then flushing out with a clean fluid.

The fluid used for flushing purposes may be water and/or air introduced from an independent source into the relative passages at a point downstream of the on-off valves, with the valves still closed, or alternatively, use can be made of the water and/or air sources that produce cooling and propulsion during normal operation, in this instance, having first opened up the respective on-off valves.

In a typical column-mounted assembly of dental surgery equipment, the supply line connecting each instrument to the main spray circuit also connects with at least one plenum chamber, able to generate a soft vacuum, the purpose of which is to prevent the instrument dripping water when switched off by deactivating the on-off valve. There may be one such chamber to serve the entire column, or one for each instrument connected to the column. In this latter instance, each plenum will generally be located downstream of the on-off valve on the relative branch of the spray circuit; accordingly, the device disclosed can be connected upstream of and used to sterilize the plenum chambers. This is an important feature, inasmuch as bacteria will inevitably tend to collect in the plenum chamber (or chambers) with continued use of the equipment, and frequent sterilization and/or disinfection is required.

Thus, an additional object of the invention is to embody a device that will also sterilize and/or disinfect, in the case in point, a single plenum chamber incorporated into the equipment column, and if not entirely eliminate, then greatly reduce the bacterial content of water drawn from the domestic system into the spray circuit ; the main object stated above is therefore flanked by the further general object of ensuring sterilization of the circuit as a whole.

The device according to the invention comprises an auxiliary sterilizer circuit connected via relative branches with the instrument supply lines of the spray circuit at points downstream of the on-off valves, which remain closed during sterilization, and this further object is achieved by connecting the plenum in series with both the sterilizer circuit and the spray circuit, and incorporating shut-off means in parallel with the spray circuit and interconnecting the sections of the instrument supply lines upstream and downstream of the on-off valves ; thus, it is the shut-off means that open to allow passage of the liquid sterilizer.

One of the advantages afforded by the invention is essentially that of the sure sterilizating action achieved, especially where sterilizer is flushed out by activating the instruments, as in this case, fluid passing through certain of the instruments can atomize internally of the collection vessel ; first to be atomized is the sterilizer, which acts on the tips of the instruments, and immediately thereafter, water from the spray circuit, which both flushes out the internal passages and floods the tips of the handgrips, as well as cleansing the entire collection vessel.

Another advantage of the invention is its efficient operation ; when the sterilizer is flushed out by activating the instruments, in fact, this has the effect of sterilizing those parts associated with though not lying directly in the path of the water, such as the turbine of the high speed drill.

A further advantage of the invention is that of dependable operation, obtained by sterilizing the instruments with the relative on-off valves in their normally-closed position, in such a way that there are no problems with sterilization failing to take effect, or with sterilizer lingering in the circuits.

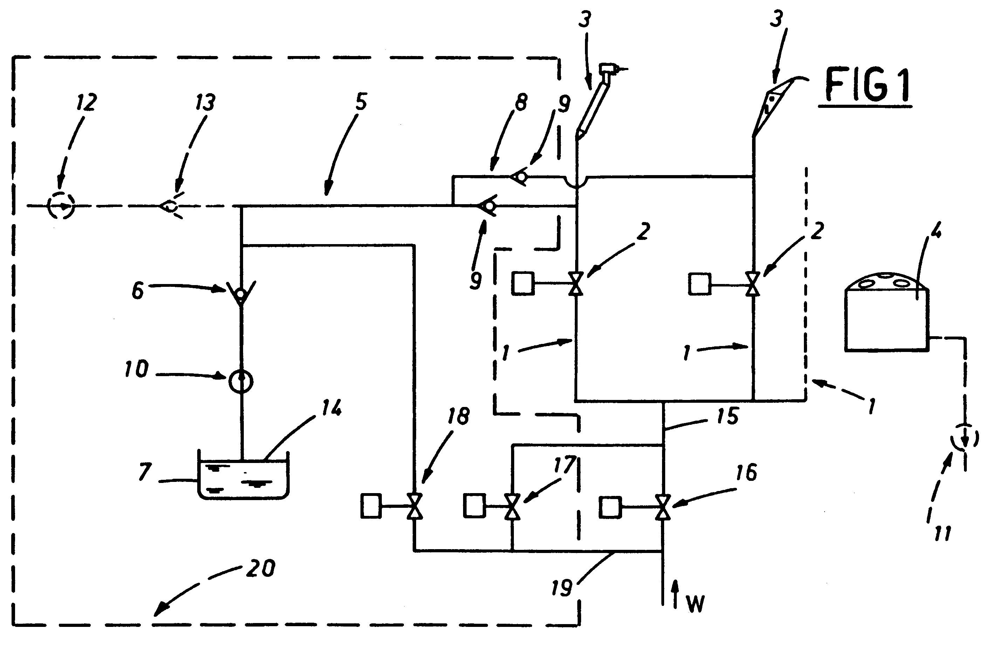

The invention will now be described in detail, by way of example, with the aid of the accompanying drawings, in which fig 1 is a schematic representation of the device according to the invention.

With reference to fig 1, the device according to the invention is associated with a given number of power driven medical instruments (in practice, the device might be incorporated permanently or used as an accessory, to equally good effect), which in the example illustrated are dental surgery instruments, denoted 3, forming part of a conventional column.

The single instruments 3 connect with fluid supply lines, denoted 1, controlled by respective on-off valves 2.

Each supply line 1 (carrying water, for example, as arrowed W in the drawings) constitutes a first branch of a main circuit 15 controlled in turn by a main shut-off valve 16. These valves 2, 16 will all be connected to a control circuit of conventional type (not illustrated) by means of which to ensure that operation of any one instrument will come about on activation of the relative on-off valve 2 together with the main valve 16. In short, to operate a given instrument 3, the relative valve 2 must be opened up, generally by lifting the handgrip from its holder in the console table of the equipment column, and the main valve 16 then opened, usually by depressing a foot pedal.

The device according to the invention essentially comprises a tank 7 containing a supply of liquid sterilizer 14, an auxiliary circuit 5 connecting with the spray circuit supply lines 1, downstream of the relative on-off valves 2, also, a vessel 4 in which the soiled sterilizer 14 is collected, and in which the instruments 3 are placed and held during the sterilization and collection operations.

The auxiliary circuit 5 affords as many second branches 8 as there are instruments, which connect with the relative supply lines 1 of the spray circuit and incorporate relative check valves 9 disallowing any return of liquids into the circuit 5; a further check valve 6 is fitted to the outlet of the feed pump 10 associated with the supply tank 7.

19 denotes a branch of the main spray circuit 15 located upstream of the main shut-off valve 16, which divides further into a by-pass, governed by a relative valve 17 and skirting the main valve 16, and a line that connects by way of a further on-off valve 18 with the auxiliary sterilizer circuit 5 at a point downstream of the pump 10. The inventive step claimed herein is reflected in the components circumscribed by the phantom line 20 in fig 1.

Referring to the device illustrated in fig 1, the process according to the invention comprises the steps of:
- removing the instruments 3 from their holders and placing them in the vessel 4;
- switching all the on-off valves 2 to 'off', such that the upstream section of the the respective instrument supply lines 1 remain closed;
- supplying sterilizer 14 from the tank 7 to the instruments;
- flushing the auxiliary circuit 5 and associated supply lines 1 with a fluid to the end of removing the soiled sterilizer 14;
- returning the instruments 3 to their holders.

The liquid sterilizer 14 may be supplied in either one of two ways, namely, activating a pump 10 that forces the liquid through the auxiliary circuit 5 as mentioned above, or connecting the vessel 4 to means 11 of generating suction (see phantom line in fig 1). In this second instance, the vessel 4 will need to be structured in such a way as to afford a fluid-tight seal once the instruments 3 have been placed in it.

In either instance, the supply tank 7 will be held at atmospheric pressure, or at all events, will not be pressurized.

Following a prescribed period commensurate with the type of germicidal treatment required, the soiled sterilizer 14 is flushed away by opening up all the on-off valves 2 (which, it will be remembered, have not been in direct contact with the sterilizer) and activating the spray circuit by way of the by-pass valve 17 so as to supply water to the instruments; in this way, one avoids operating the foot pedal to open up the main shut-off valve 16. At the same time, water is sent into the part of the auxiliary circuit 5 downstream of the pump check valve 6 by opening the valve denoted 18.

In a preferred, but by no means definitive version of the process and the device, the flushing step is accomplished by activating all the instruments 3 in the vessel 4 at once (that is, switching on all the relative supplies of fluid). This constitutes a particularly effective expedient, since there will be an initial explusion, by all the instruments 3 together, of sterilizer 14 still in the parts of the two circuits as mentioned above, i.e. 1, 5, 8, following which, these same parts will be flooded with water.

Given that certain dental surgery instruments 3 are designed to emit an atomized spray during normal operation, these will at first discharge an almost totally clean, atomized spray of sterilizer that is able to fill the collection vessel 4. The effect of such atomization is to perform a cleansing action on the tips of the instruments, as long as there is sterilizer 14 still in the circuits 1, 5, 8, and when clean water emerges thereafter, to effect a thorough rinse of the tips.

It is especially advantageous to flush out the sterilizer by full activation of the instruments 3 in view of the additional fact that those parts of the instruments connected with the spray circuit 1 though not directly invested by the spray, such as certain parts of the high speed drill, will be subjected to the action of the sterilizer 14 and then flooded with water; accordingly, each single instrument 3 emerges from the vessel 4 thoroughly sterilized and rinsed clean.

In an alternative version of the process and the device, the soiled sterilizer 14 is flushed out by leaving the on-off valves 2 closed and blasting the auxiliary circuit 5 downstream of the pump check valve 6 with compressed air.

In this instance, the device will comprise a source of compressed air 12 (normally speaking, that of the surgery equipment column itself) and a check valve 13 (illustrated in phantom lines in both of the drawings), located immediately downstream of the pump 10 and its check valve 6, which serves to prevent any return of fluid, air or sterilizer when the source 12 is switched off.

This method of implementing the flushing step is especially practical, as the sterilizer 14 can be removed using a fluid that requires no conditioning and, as a result, is most economical.

The further option exists of drawing in sterilizer from a supply held in the vessel 4, in which case flow through the auxiliary circuit 5 is reversed, with the pump 10 generating negative rather than positive pressure. The instruments 3 having been placed in the vessel 4, immersed in the sterilizer, and the pump 10 switched on, the sterilizer will be drawn into the relative parts of the circuits 1, 8 and 5, discharging ultimately into the tank 7. The check valves, if incorporated, would be inverted.

Where this version of the process is adopted, one has the additional advantage of a 'total immersion' of those parts of the instrument 3 that require sterilization, such as is bound to enhance the efficiency of the operation. The subsequent flush would be effected in the manner already described.

The foregoing description assumes, for example, that the surgery equipment incorporates one plenum chamber (not illustrated) for each instrument 3 served by a supply line 1, located upstream of the instrument but downstream of the relative branch 8 of the auxiliary circuit 5.

## Claims

1. A process for sterilization of the spray and/or air supply lines to medical instruments, in particular dental surgery instruments, connected to a main spray circuit (15) via supply lines (1) each constituting one first branch of said main circuit (15) which is controlled by a main shut-off valve (16), each of said supply line (1) being controlled by a on-off valves (2) defining a downstream section and an upstream section, comprising the steps of:
- removing the instruments (3) from their holders and placing them in a collection vessel (4);
- switching all the on-off valves (2) to the 'off' position, so that each instrument supply line (1) from the spray circuit remains closed at least immediately upstream on the relative valve (2) in correspondence of said upstream sections;
- supplying liquid sterilizer (14) to the supply lines (1) only in correspondence of said downstream sections by way of second branches (8) of an independent circuit (5), each of said branches (8) connecting with each supply line at a point downstream of the relative on-off valve (2);
- directing a fluid into the supply line (1) to the end of removing the liquid sterilizer (14);
- returning the instruments (3) to their holders.

2. A process for sterilization of the spray and/or air supply lines to medical instruments, in particular dental surgery instruments, connected to a main spray circuit (15) via supply lines (1), each constituting one first branch of said main circuit (15) which is controlled by a main shut-off valve (16) each of said supply line (1) being controlled by a on-off valves (2) defining a downstream section and an upstream section, comprising the steps of:
- removing the instruments (3) from their holders and placing them in a vessel (4) containing liquid sterilizer, in such a way that the instruments are immersed in the liquid;
- switching all the on-off valves (2) to 'off', in such a way that each supply line (1) from the spray cicuit to the instrument remains closed at least immediately upstream of the relative valve (2) in correspondence of said upstream sections;
- activating suctions means (10), connected via an independent circuit (5) with the instrument supply lines (1) at points [of said free valve sections] downstream of the respective on-off valves (2) to draw in the liquid sterilizer (14) from the vessel;
- directing a fluid into the supply lines (1), to the end of removing the liquid sterilizer (14);
- returning the instruments (3) to their holders.

3. A process as in claim 1 or claim 2, wherein the steps of directing a fluid into the instrument supply lines (1) to remove the liquid sterilizer (14) consists in opening up all the relative on-off valves (2) and activating the means by which fluid is supplied to the selfsame supply lines (1) during normal operation.

4. A process as in claim 3, wherein the step of directing a fluid into the instrument supply lines (1) to remove the liquid sterilizer (14) consists in opening up all the relative on-off valves (2) and activating all the fluid power sources to which the instruments (3) are connected.

5. A process as in claim 1 or claim 2, wherein the step of directing a fluid into the instrument supply lines (1) to remove the liquid sterilizer (14) consists in directing compressed air into the independent auxiliary circuit (5).

6. A device for sterilization of the spray and/or air supply lines to medical instruments, in particular dental surgery instruments, connected to a main spray circuit (15) via supply lines each constituting a first branch of a main circuit (15) which is controlled by a main shut-off valve (16) (1), each of said supply line 1 being controlled by an on-off valve (2), comprising:
- a tank (7) containing liquid sterilizer (14);
- an independent auxiliary circuit (5) departing from the tank (7) and connecting by way of second branches (8) with each supply line (1) at a point downstream of the respective on-off valve (2);
- a vessel (4) in which the liquid sterilizer (14) is collected, and in which the instruments (3) can be placed and held;
- means for effecting transfer of the sterilizer.

7. A device as in claim 6, wherein the transfer means consist in a pump (10) located between the tank (7) and the independent auxiliary circuit (5), by which the liquid sterilizer (14) is directed into the auxiliary circuit (5), and each branch (8) of the circuit (5) connecting with a relative instrument supply line (1) incorporates a respective check valve (9) serving to prevent any return of fluid in the direction of the tank (7).

8. A device as in claim 6, wherein the transfer means consist in a pump (11) located downstream of and serving to draw liquid from the vessel (4), and the vessel (4) is embodied so as to remain fluid-tight when instruments are placed inside it.

9. A device as in claim 6, further comprising:
- a source of compressed air (12) connected to the auxiliary sterilizer circuit (5);
- a first check valve (6) located between the air source (12) and the auxiliary circuit (5), serving to prevent any return of compressed air in the direction of the tank (7);
- a second check valve (13) located between the air source (12) and the auxiliary circuit (5), serving to prevent any return of liquid sterilizer (14) in the direction of the air source (12) during the step in which transfer is effected to the supply lines (1).

10. A device as in claim 6, associated with surgery equipment in which the main spray circuit (15) is controlled by a main shut-off valve (16) located upstream of the on-off valves (2), wherein the spray circuit (15) is branched at a point upstream of the main shut-off valve (16) in order to create a by-pass loop around the main shut-off valve (16) and to enable connection with the auxiliary circuit (5) at a point downstream of the pump (10), and wherein the by-pass loop and the connection with the auxiliary circuit are controlled by respective on-off valves (17, 18) that operate to open during the step in which a fluid is directed into the supply lines (1) to the end of removing the liquid sterilizer.

11. A device as in claim 6, wherein the pressure level in the tank (7) containing liquid sterilizer (14) is substantially atmospheric.

12. A device for sterilization of the spray and/or air supply lines to medical instruments, in particular dental surgery instruments, connected to a main spray circuit via supply lines (1) controlled by relative on-off valves (2), in accordance with the process as in claim 2,
characterized in that it comprises:
- a vessel (4) containing liquid sterilizer (14), in which the instruments (3) can be placed and held, immersed in the sterilizer;
- a tank (7) into which the liquid sterilizer (14) is exhausted;
- an independent circuit (5), connecting with each instrument supply line (1) at a point downstream of the relative on-off valve (2) and incorporating suction means (10) designed to implement the step of drawing in the liquid sterilizer (14) from the vessel (4).

## Patentansprüche

1. Verfahren zur Sterilisation der Zerstäuber- und/oder der Luftzufuhrleitungen von medizinischen Instrumenten, insbesondere von zahnärztlichen Instrumenten, die an einen Hauptzerstäuberkreis (15) angeschlossen sind, und zwar über Zuführleitungen (1), von denen jede eine erste Abzweigung des genannten Hauptzerstäuberkreises (15) bildet, welcher durch ein Hauptabsperrventil (16) gesteuert wird, wobei jede der genannten Zuführleitungen (1) von Absperrventilen (2) gesteuert wird, die einen stromabwärts gerichteten Abschnitt und einen stromaufwärts gerichteten Abschnitt beschreiben, enthaltend die folgenden Phasen:
- Entnahme der Instrumente (3) aus deren Halterungen und Hineinlegen derselben in einen Sammelbehälter (4);
- Umstellen aller Absperrventile (2) auf die Position "Aus", so dass jede Instrumenten-Zuführleitung (1) vom Zerstäuberkreis her geschlossen bleibt, zumindest unmittelbar oberhalb des entsprechenden Ventils (2) und entsprechend dem stromaufwärts gerichteten Abschnitt;
- Zufuhr einer Sterilisierflüssigkeit (14) in die Zuführleitungen (1) nur im Bereich der genannten stromabwärts gerichteten Abschnitte mit Hilfe von zweiten Abzweigungen (8) an einen unabhängigen Kreis (5), wobei jede der genannten Abzweigungen (8) mit einer jeden Zuführleitung an einem Punkt unterhalb des entsprechenden Absperrventils (2) verbunden ist;
- Eingabe einer Flüssigkeit in die Zuführleitung (1) zu dem Zweck, die Sterilisierflüssigkeit (14) aus dieser herauszuspülen;
- Wiederanordnung der Instrumente (3) in ihren Halterungen.

2. Verfahren zur Sterilisation der Zerstäuber- und/oder der Luftzufuhrleitungen von medizinischen Instrumenten, insbesondere von zahnärztlichen Instrumenten, die an einen Hauptzerstäuberkreis (15) angeschlossen sind, und zwar über Zuführleitungen (1), von denen jede eine erste Abzweigung des genannten Hauptzerstäuberkreises (15) bildet, welcher durch ein Hauptabsperrventil (16) gesteuert wird, wobei jede der genannten Zuführleitungen (1) von Absperrventilen (2) gesteuert wird, die einen stromabwärts gerichteten Abschnitt und einen stromaufwärts gerichteten Abschnitt beschreiben, enthaltend die folgenden Phasen:
- Entnahme der Instrumente (3) aus deren Halterungen und Hineinlegen derselben in einen Behälter (4), der eine Sterilisierflüssigkeit enthält, und zwar auf solche Weise, dass die Instrumente in die Flüssigkeit eingetaucht sind;
- Umstellen aller Absperrventile (2) auf die Position "Aus", so dass jede Zuführleitung (1) vom Zerstäuberkreis zum Instrument geschlossen bleibt, zumindest unmittelbar oberhalb des entsprechenden Ventils (2) und entsprechend dem stromaufwärts gerichteten Abschnitt;
- Aktivierung von Absaugmitteln (10), die über einen unabhängigen Kreis (5) an die Zuführleitungen (1) zu den Instrumenten angeschlossen sind, und zwar an Punkten [der genannten freien Ventilabschnitte] unterhalb der entsprechenden Absperrventile (2), um die Sterilisierflüssigkeit (14) aus dem Behälter abzusaugen;
- Eingabe einer Flüssigkeit in die Zuführleitungen (1) zu dem Zweck, die Sterilisierflüssigkeit (14) aus dieser herauszuspülen;
- Wiederanordnung der Instrumente (3) in ihren Halterungen.

3. Verfahren nach Patentanspruch 1 oder 2, bei welchem die Phasen des Einleitens einer Flüssigkeit in die Zuführleitungen (1) der Instrumente zum Herausspülen der Sterilisierflüssigkeit darin bestehen, dass alle entsprechenden Absperrventile (2) geöffnet und die Mittel aktiviert werden, durch welche Flüssigkeit während des normalen Betriebes an dieselben Zuführleitungen (1) geführt wird.

4. Verfahren nach Patentanspruch 3, bei welchem die Phasen des Einleitens einer Flüssigkeit in die Zuführleitungen (1) der Instrumente zum Herausspülen der Sterilisierflüssigkeit darin bestehen, dass alle entsprechenden Absperrventile (2) geöffnet und alle die Flüssigkeitsdruckquellen aktiviert werden, an welche die Instrumente (3) angeschlossen sind.

5. Verfahren nach Patentanspruch 1 oder 2, bei welchem die Phase des Einleitens einer Flüssigkeit in die Zuführleitungen (1) der Instrumente zum Herausspülen der Sterilisierflüssigkeit (14) darin bestehen, dass Druckluft in den unabhängigen Hilfskreis (5) eingegeben wird.

6. Vorrichtung zur Sterilisation der Zerstäuber- und/oder der Luftzufuhrleitungen von medizinischen Instrumenten, insbesondere von zahnärztlichen Instrumenten, angeschlossen an einen Hauptzerstäuberkreis (15), und zwar über Zuführleitungen (1), von denen jede eine erste Abzweigung des genannten Hauptzerstäuberkreises (15) bildet, welcher durch ein Hauptabsperrventil (16) gesteuert wird, wobei jede der genannten Zuführleitungen (1) von Absperrventilen (2) gesteuert wird, enthaltend:
- einen Behälter (7), welcher eine Sterilisierflüssigkeit (14) enthält;
- einen unabhängigen Hilfskreis (5), welcher von dem Behälter (7) ausgeht und über zweite Abzweigungen (8) mit einer jeden Zuführleitung (1) an einem Punkt unterhalb des entsprechenden Absperrventils (2) verbunden ist;
- einen Sammelbehälter (4), in welchem die Sterilisierflüssigkeit (14) aufgefangen wird, und in welchen die Instrumente (3) hineingelegt und darin gehalten werden können;
- Mittel zum Weiterleiten des Sterilisiermittels.

7. Vorrichtung nach Patentanspruch 6, bei welcher die Mittel zum Weiterleiten aus einer Pumpe (10) bestehen, die zwischen dem genannten Behälter (7) und dem unabhängigen Hilfskreis (5) angeordnet ist, und durch welche die Sterilisierflüssigkeit (14) in den Hilfskreis (5) geleitet wird, wobei jede Abzweigung (8) des mit einer entsprechenden Zuführleitung (1) der Instrumente verbundenen Kreises (5) ein entsprechendes Rückschlagventil (9) enthält, das dazu dient, jeden Rückfluß der Flüssigkeit in Richtung des Behälters (7) zu verhindern.

8. Vorrichtung nach Patentanspruch 6, bei welcher die Mittel zum Weiterleiten aus einer Pumpe (11) bestehen, welche unterhalb des Sammelbehälters (4) angeordnet ist und dazu dient, die Flüssigkeit aus dem Sammelbehälter (4) abzuziehen, und bei welcher der Sammelbehälter (4) auf solche Weise ausgelegt ist, dass er hermetisch verschlossen bleibt, wenn die Instrumente in diesem angeordnet sind.

9. Vorrichtung nach Patentanspruch 6, weiter enthaltend:
- eine Druckluftquelle (12), die an den Hilfs-Sterilisierkreis (5) angeschlossen ist;
- ein erstes Rückschlagventil (6), angeordnet zwischen der Druckluftquelle (12) und dem Hilfskreis (5), welches dazu dient, jeden Rückstrom von Druckluft in Richtung des Behälters (7) zu verhindern;
- ein zweites Rückschlagventil (13), angeordnet zwischen der Druckluftquelle (12) und dem Hilfskreis (5), welches dazu dient, jeden Rücklauf von Sterilisierflüssigkeit (14) in Richtung der Druckluftquelle (12) zu verhindern, und zwar während der Phase, in das Weiterleiten zu den Zuführleitungen (1) erfolgt.

10. Vorrichtung nach Patentanspruch 6, verbunden mit einer medizinischen Anlage, bei welcher der Hauptzerstäuberkreis (15) durch ein Absperrventil (16) gesteuert wird, welches oberhalb des Absperrventils (2) angeordnet ist, bei welcher der Zerstäuberkreis (15) von einem Punkt oberhalb des Hauptabsperrventils (16) abgezweigt ist, so dass ein Bypass um das Hauptabsperrventil (16) herum geschaffen und der Anschluss an den Hilfskreis (5) an einem Punkt unterhalb der Pumpe (10) ermöglicht wird, und bei welcher der Bypass und der Anschluss an den Hilfskreis durch entsprechende Absperrventile (17, 18) gesteuert werden, die das Öffnen während der Phase besorgen, in welcher eine Flüssigkeit mit dem Ziel in die Zuführleitungen (1) eingeführt wird, die Sterilisierflüssigkeit zu entfernen.

11. Vorrichtung nach Patentanspruch 6, bei welcher der Druckpegel in dem die Sterilisierflüssigkeit (14) enthaltenden Behälter (7) im wesentlichen dem atmosphärischen Druck entspricht.

12. Vorrichtung zur Sterilisation der Zerstäuber- und/oder der Luftzufuhrleitungen von medizinischen Instrumenten, insbesondere von zahnärztlichen Instrumenten, angeschlossen an einen Hauptzerstäuberkreis, und zwar über Zuführleitungen (1), welche in Übereinstimmung mit dem Verfahren nach Patentanspruch 2 durch entsprechende Absperrventile (2) gesteuert werden, enthaltend:
- einen Sammelbehälter (4) zur Aufnahme der Sterilisierflüssigkeit (14), in welchen die Instrumente (3) hineingelegt und in die Sterilisierflüssigkeit eingetaucht gehalten werden können;
- einen Behälter (7), in welchen die Sterilisierflüssigkeit (14) entleert wird;
- einen unabhängigen Kreis (5), der mit jeder Zuführleitung (1) der Instrumente an einem Punkt unterhalb des entsprechenden Absperrventils (2) verbunden ist und Absaugmittel (10) enthält, die dazu bestimmt sind, die Phase des Absaugens der Sterilisierflüssigkeit (14) aus dem Sammelbehälter (4) zu besorgen.

## Revendications

1. Procédé pour la stérilisation du pulvérisateur et/ou de la conduite d'air des instruments médicaux, en particulier des instruments de chirurgie dentaire, reliés à un circuit principal du pulvérisateur (15) à travers les conduites d'amenée (1), chacune constituant une première ramification dudit circuit principal (15) qui est contrôlé par une soupape principale d'isolement (16), chacune desdites conduites d'amenée (1) étant commandée par une soupape de marche-arrêt (2) définissant une section en aval et une section en amont, comportant les étapes de:
- enlever les instruments (3) de leurs supports et les placer dans un récipient collecteur (4);
- porter toutes les soupapes de marche-arrêt (2) à la position d'arrêt, de manière que chaque conduite d'amenée (1) des instruments en provenance du circuit du pulvérisateur reste fermée au moins immédiatement en amont de la respective soupape (2), en correspondance desdites sections en amont;
- n'alimenter les conduites d'amenée (1) en liquide stérilisant (14) qu'en correspondance desdites sections en aval par l'intermédiaire de deuxièmes ramifications (8) d'un circuit indépendant (5), chacune desdites ramifications (8) étant en communication avec chaque conduite d'amenée à un endroit à l'aval de la respective soupape de marche-arrêt (2);
- diriger un fluide dans la conduite d'amenée (1) dans le but de faire sortir le liquide stérilisant (14);
- remettre en place les instruments (3) sur leurs supports.

2. Procédé pour la stérilisation du pulvérisateur et/ou de la conduite d'air des instruments médicaux, en particulier des instruments de chirurgie dentaire, reliés à un circuit principal du pulvérisateur (15), à travers les conduites d'amenée (1), chacune constituant une première ramification dudit circuit principal (15) qui est contrôlé par une soupape principale d'isolement (16), chacune desdites conduites d'amenée (1) étant commandée par une soupape de marche-arrêt (2) définissant une section en aval et une section en amont, comportant les étapes de:
- enlever les instruments (3) de leurs supports et les placer dans un récipient (4) contenant du liquide stérilisant, de telle sorte que les instruments soient plongés dans le liquide;
- porter toutes les soupapes de marche-arrêt (2) à la position d'arrêt, de telle sorte que chaque conduite d'amenée (1) depuis le circuit du pulvérisateur jusqu'à l'instrument reste fermée au moins immédiatement en amont de la soupape respective (2) en correspondance desdites sections en amont;
- activer des moyens d'aspiration (10), reliés par l'intermédiaire d'un circuit indépendant (5) aux conduites d'amenée (1) des instruments à des emplacements [desdites sections de soupapes libres] en aval des respectives soupapes de marche-arrêt (2), en vue d'aspirer du récipient le liquide stérilisant (14);
- diriger un fluide dans les conduites d'amenée (1), dans le but de faire sortir le liquide stérilisant (14);
- remettre en place les instruments (3) sur leurs supports.

3. Procédé selon la revendication 1 ou la revendication 2, dans lequel les étapes de diriger un fluide dans les conduites d'amenée (1) des instruments en vue de faire sortir le liquide stérilisant (14) consistent à ouvrir toutes les soupapes de marche-arrêt (2) respectives et activer les moyens par lesquels les mêmes conduites d'amenée (1) sont alimentées en fluide pendant le fonctionnement normal.

4. Procédé selon la revendication 3, dans lequel l'étape de diriger un fluide dans les conduites d'amenée (1) des instruments pour faire sortir le liquide stérilisant (14) consiste à ouvrir toutes les soupapes de marche-arrê (2) respectives et à activer toutes les sources d'énergie auxquelles les instruments (3) sont reliés.

5. Procédé selon la revendication 1 ou la revendication 2, dans lequel l'étape de diriger un fluide dans les conduites d'amenée (1) des instruments en vue de faire sortir le liquide stérilisant (14) consiste à envoyer de l'air comprimé dans le circuit auxiliaire indépendant (5).

6. Dispositif pour la stérilisation du pulvérisateur et/ou de la conduite d'air des instruments médicaux, en particulier des instruments de chirurgie dentaire, reliés à un circuit principal du pulvérisateur (15) par l'intermédiaire des conduites d'amenée (1) dont chacune constitue une première ramification d'un circuit principal (15) qui est contrôlé par une soupape principale d'isolement (16), chacune desdites conduites d'amenée (1) étant commandée par une soupape de marche-arrêt (2), comportant:
- un réservoir (7) contenant du liquide stérilisant (14);
- un circuit auxiliaire indépendant (5) qui part du réservoir (7) et est en communication, par des deuxièmes ramifications (8), avec chaque conduite d'amenée (1) à un endroit en aval de la soupape de marche-arrêt (2) respective;
- un récipient (4) dans lequel est recueilli le liquide stérilisant (14) et dans lequel peuvent être disposés et maintenus les instruments (3);
- des moyens pour effectuer le transfert du liquide stérilisant.

7. Dispositif selon la revendication 6, dans lequel les moyens de transfert consistent en une pompe (10) située entre le réservoir (7) et le circuit auxiliaire indépendant (5), par laquelle le liquide stérilisant (14) est dirigé dans le circuit auxiliaire (5) et chaque ramification (8) du circuit (5) qui est en communication avec une respective conduite d'amenée (1) des instruments incorpore une soupape de retenue (9) respective qui sert à empêcher que le fluide retourne vers le réservoir (7).

8. Dispositif selon la revendication 6, dans lequel les moyens de transfert consistent en une pompe (11) disposée en aval du récipient (4) et servant à aspirer le liquide de ce dernier, et le récipient (4) est réalisé de telle sorte qu'il reste étanche aux fluides quand les instruments sont placés à son intérieur.

9. Dispositif selon la revendication 6, comportant en outre:
- une source d'air comprimé (12) reliée au circuit auxiliaire de liquide stérilisant (5);
- une première soupape de retenue (6) disposée entre la source d'air (12) et le circuit auxiliaire (5), laquelle sert à empêcher le retour de l'air comprimé dans la direction du réservoir (7);
- une deuxième soupape de retenue (13) disposée entre la source d'air (12) et le circuit auxiliaire (5), laquelle sert à empêcher le retour du liquide stérilisant (14) dans la direction de la source d'air (12), lors de l'étape pendant laquelle il y a le transfert aux conduites d'amenée (1).

10. Dispositif selon la revendication 6, associé à l'appareillage de chirurgie dentaire dans lequel le circuit principal du pulvérisateur (15) est commandé par une soupape principale d'isolement (16) située en amont des soupapes de marche-arrêt (2), dans lequel le circuit du pulvérisateur (15) est ramifié à un emplacement en amont de la soupape principale d'isolement (16) dans le but de créer une voie de dérivation autour de la soupape d'isolement principale (16) et de permettre la liaison au circuit auxiliaire (5) à un endroit en aval de la pompe (10), et dans lequel la voie de dérivation et la liaison au circuit auxiliaire sont commandées par des soupapes de marche-arrêt respectives (17, 18) qui sont activées de manière à être ouvertes lors de l'étape pendant laquelle un fluide est dirigé vers les conduites d'amenée (1), dans le but de faire sortir le liquide stérilisant.

11. Dispositif selon la revendication 6, dans lequel la pression dans le réservoir (7) contenant le liquide stérilisant (14) est sensiblement celle atmosphérique.

12. Dispositif pour la stérilisation du pulvérisateur et/ou de la conduite d'air des instruments médicaux, en particulier des instruments de chirurgie dentaire, reliés à un circuit principal du pulvérisateur au travers des conduites d'amenée (1) commandées par des soupapes de marche-arrêt respectives (2), selon le procédé de la revendication 2,
caractérisé en ce qu'il comporte:
- un récipient (4) contenant le liquide stérilisant (14), dans lequel peuvent être mis et maintenus les instruments (3), plongés dans le liquide stérilisant;
- un réservoir (7) dans lequel est déchargé le liquide stérilisant (14);
- un circuit indépendant (5), qui est en communication avec chaque conduite d'amenée (1) des instruments à un endroit en aval de la soupape de marche-arrêt respective (2) et incorpore des moyens d'aspiration (10) destinés à réaliser l'étape d'aspiration du liquide stérilisant (14) du récipient (4).
